# EUROPÄISCHE PATENTSCHRIFT

(11) **EP 4 060 190 B1**
(45) Veröffentlichungstag und Bekanntmachung des Hinweises auf die Patenterteilung: **04.09.2024**
(21) Anmeldenummer: 22161707.9
(22) Anmeldetag: 11.03.2022
(51) Int. Cl.: F03G 7/06, B60N 2/90, G01F 1/684, G01F 1/69, G01F 15/00

(54) **SCHALTUNGSANORDNUNG UND VERFAHREN ZUM ANSTEUERN MINDESTENS EINES VENTILS, VENTIL, VENTILANORDNUNG UND SITZKOMFORTSYSTEM**
CIRCUIT ASSEMBLY AND METHOD FOR CONTROLLING AT LEAST ONE VALVE, VALVE, VALVE ARRANGEMENT AND SEATING COMFORT SYSTEM
AGENCEMENT DE CIRCUIT ET PROCÉDÉ DE COMMANDE D'AU MOINS UNE SOUPAPE, SOUPAPE, AGENCEMENT DE SOUPAPE ET SYSTÈME DE CONFORT DE SIÈGE

(30) Priorität: 15.03.2021 DE 102021106252
(43) Veröffentlichungstag der Anmeldung: 21.09.2022
(73) Patentinhaber: Gentherm Präzision SE, 91757 Treuchtlingen (DE)
(72) Erfinder: Dörfler, Erich, 86899 Landsberg (DE); Mitzler, Matthias, 91757 Graben (DE)
(74) Vertreter: Schlögl, Markus

(56) Entgegenhaltungen:
- EP-A2- 0 940 742
- DE-A1- 102009 035 617
- DE-A1- 102015 214 569

## Beschreibung

Die Erfindung betrifft ein Sitzkomfortsystem umfassend ein oder mehrere Luftkissen, wobei der Füllstand jedes Luftkissens über zumindest ein Ventil steuerbar ist. Das Sitzkomfortsystem umfasst ferner ein oder mehrere Schaltungsanordnungen zur Ansteuerung des oder der Ventile. Das mindestens eine Ventil umfasst zumindest einen Aktuator mit zumindest einem Stellelement, wobei das Stellelement zumindest zwischen einer ersten Stellung und einer zweiten Stellung verstellbar ist. Die Schaltungsanordnung weist zumindest eine Treibereinheit zum Betätigen des Aktuators und eine Steuereinheit zum Steuern der Treibereinheit auf.

Nach dem Stand der Technik ist zum Beispiel aus der DE 10 2017 112 803 A1 eine Schaltungsanordnung zur Ansteuerung eines Systems, insbesondere einer Lordosenstütze mit zumindest zwei Luftkissen bekannt. Die bekannte Schaltung ist somit für ein Sitzkomfortsystem geeignet.

Ein Sitzkomfortsystem ist ein System für eine Sitzkomfortfunktion eines Sitzes, beispielsweise eines Fahrzeugsitzes. Beispielsweise kann ein derartiges Sitzkomfortsystemmit eine pneumatische Lordosenstützvorrichtung und/oder eine Massagevorrichtung für einen Sitz umfassen. Ein Sitzkomfortsystem umfasst typischerweise eine Mehrzahl von Luftkissen, welche gefüllt und mit einem gewünschten, gegebenenfalls zeitlich veränderten Druck beaufschlagt werden können. Dazu umfasst das Sitzkomfortsystem eine Steuereinheit, eine Pumpe zum Beaufschlagen der Luftkissen mit Druckluft und eine Mehrzahl von Ventilen, wobei zweckmäßigerweise jedem Luftkissen zumindest ein Ventil zugeordnet ist. Jedes dieser Ventile kann einen Aktuator mit einem SMA-Element (SMA: shape memory alloy - Formgedächtnislegierung), also einem aus einer Formgedächtnislegierung bestehenden Element umfassen, welches je nach Bestromung das Ventil in einen geöffneten, teilgeöffneten oder geschlossenen Funktionszustand versetzt. Üblicherweise handelt es sich bei dem SMA-Element um einen SMA-Draht. Die zugeführte Leistung muss üblicherweise in einem sehr eng definierten Bereich gehalten werden, um eine zuverlässige Aktivierung des Aktuators zu gewährleisten und dennoch eine thermische Überlastung und somit eine dauerhafte Schädigung des SMA-Elementes zu vermeiden. Somit sind der Steuereinheit zweckmäßigerweise Sensoren zur Strom-, Spannungs- und/oder Temperaturüberwachung zugeordnet.

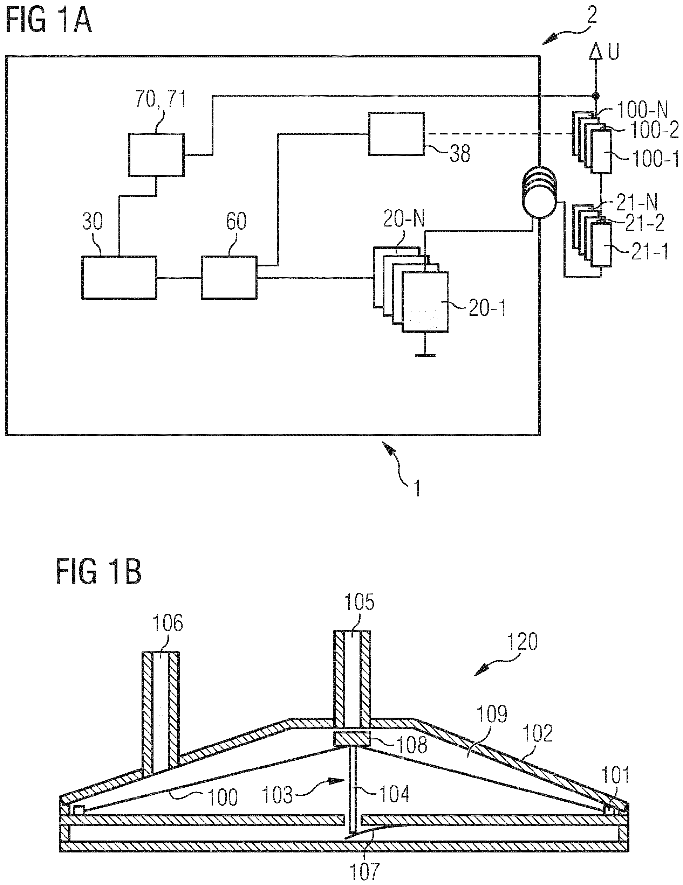

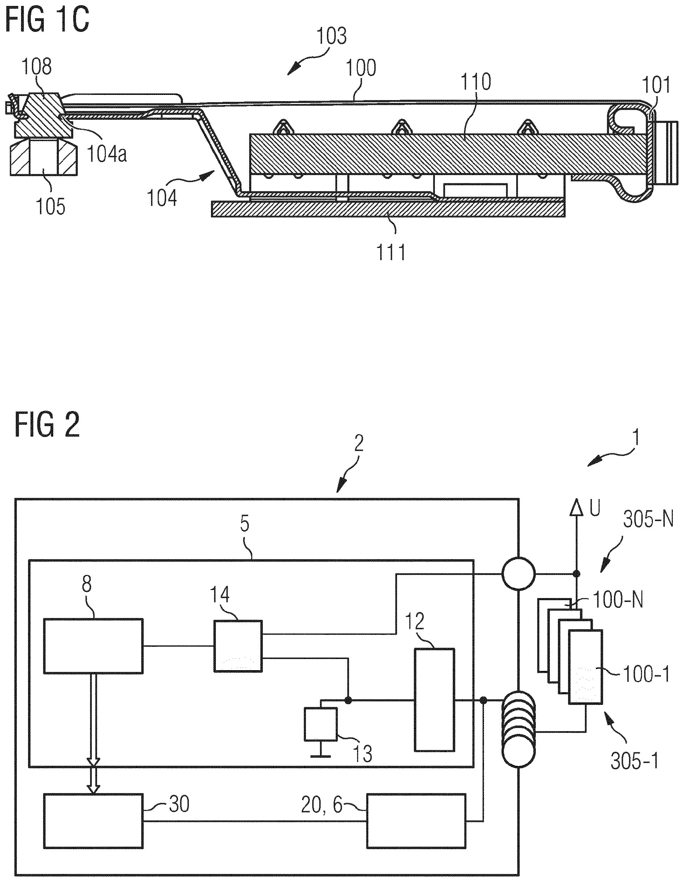

Fig. 1A, Fig. 1B und Fig. 1C zeigen eine Schaltungsanordnung mehrerer Ventile 120 eines Sitzkomfortsystem 2 nach dem Stand der Technik. Die Schaltungsanordnung 1 ist zur beispielsweise zur Ansteuerung der Ventile eines Sitzkomfortsystems 2 umfassend eine Lordosenstützvorrichtung mit zumindest zwei Luftkissen (nicht dargestellt) geeignet. Ein solches Sitzkomfortsystem 2 umfasst somit zumindest zwei Luftkissen, die zumindest ein Ventil 120 (siehe Fig. 1B, Fig. 1C), insbesondere zumindest je ein Ventil 120, umfassen. Ein solches Ventil 120 umfasst, wie beispielhaft in Fig. 1B dargestellt, ein Ventilgehäuse 102 und einen Aktuator 103. Das Ventilgehäuse 102 umfasst eine erste Öffnung 105 und eine zweite Öffnung 106. Das Gehäuse umschließt einen Ventilraum 109. Der Aktuator 103 umfasst ein SMA-Element 100, welches als V-förmig angeordneter SMA-Draht ausgeführt ist, und ein mit dem SMA-Element 100 bewegbares Stellelement 104, welches mit einem Dichtelement 108 versehen ist, um die erste Öffnung 105 wahlweise zu öffnen oder zu verschließen. Die erste Öffnung 105 ist in einer ersten Stellung des Stellelements 104 geöffnet und in einer zweiten Stellung des Stellelements 104 geschlossen.

Aus der WO 2005/026592 A2 ist bekannt, dass ein solches Ventil einen Endschalter 107 aufweisen kann, welcher bei Erreichen der zweiten Stellung geschlossen wird. Mittels dieses Endschalters kann eine dem SMA-Element 100 zugeführte Heizleistung teilweise oder vollständig reduziert werden. Weiterhin ist aus der

WO 2005/026592 A2 eine Schaltung mit einem Temperatursensor bekannt, die die Heizleistung an die Umgebungstemperatur anpasst.

Alternativ zu dem in Fig. 1B gezeigten Aktuator mit einen V-förmigen SMA-Element ist ein Aktuator 103 mit einem linearen oder U-förmigen SMA-Element 100 bekannt, bei dem das Stellelement 104 z.B. aus einer Blattfeder gebildet sein kann, an dessen ersten Ende das SMA-Element 100 angreift. Eine derartige Ausführungsform ist in Fig.1C gezeigt. Das Dichtelement 108 kann dabei in einem Durchloch 104a des Stellelements 104 an dem ersten Ende des Stellelements 104 gehalten sein. Das Stellelement 104 ist im gezeigten Beispiel zwischen einer Grundplatte 111 und einer Platine oder Leiterplatte 110 mit seinem anderen Ende angeordnet, wobei mittels eines Crimps 101 das SMA-Element 100 gehalten und kontaktiert werden kann.

Die in Fig. 1A gezeigte Schaltungsanordnung ist geeignet, mehrerer SMA-Elemente 100-1 bis 100-N zu bestromen und zu schalten. Die SMA-Elemente 100-1 bis 100-N sind mit einer mit dem Pfeil dargestellten Spannungsquelle U verbunden. Dazu umfasst die Schaltungsanordnung 1 eine Steuereinheit 30. Weiterhin kann ein Temperatursensor 70 zum Messen der Umgebungstemperatur der SMA-Elemente 100 und/oder ein Spannungssensor 71 vorhanden sein. Die Steuereinheit 30 ist mit einer Pulsweitenmodulationsvorrichtung 60 verbunden. Mittels der Pulsweitenmodulationsvorrichtung 60 kann eine Ansteuerung der SMA-Elemente 100-1 bis 100-N mittels Pulsweitenmodulation erfolgen, wobei in Abhängigkeit von der gemessenen Versorgungsspannung und Temperatur der Duty-Cycle der Pulsweitenmodulation, also das Verhältnis von Pulsweite zu Periodendauer, eingestellt wird. Eine Schaltung für eine Pulsweitenmodulation ist beispielsweise aus der DE 10 2017 112 803 A1 bekannt. Mittels des jeweiligen Treibers 20-1 bis 20-N kann das zugeordnete SMA-Element 100-1 bis 100-N nacheinander bestromt werden. Zur Vermeidung von Stromspitzen können Vorwiderstände 21-1 bis 21-N vorhanden sein. Weiterhin kann die Schaltungsanordnung auch einen Endschalter oder eine Feedback-Vorrichtung 38 umfassen. Die Feedback-Vorrichtung 38 ist entweder wie dargestellt mit der Pulsweitenmodulationsvorrichtung 60 oder alternativ mit der Steuereinheit 30 verbunden und ist geeignet, das Erreichen einer Endposition des Aktuators, welches mechanisch detektiert wird, an die Pulsweitenmodulationsvorrichtung 60 oder die Steuereinheit 30 zu melden.

Aus der DE 10 2016 225 519 A1 ist ein pneumatisches Ventil mit einem Aktuator und beweglichen Absperrelementen bekannt, wobei der Aktuator durch ein mittels elektrischer Heizleistung verformbares SMA-Element betätigt wird. Zur Betätigung des Aktuators wird dem SMA-Element eine elektrische Heizleistung zugeführt, worauf sich das SMA-Element in an sich bekannter Weise verformt und hierdurch eine vorgegebene Bewegung des Absperrelements zum Öffnen oder Schließen eines Luftanschlusses bewirkt wird. Die Verformung des SMA-Elements wird bei Beendigung der Zufuhr der elektrischen Heizleistung rückgängig gemacht, wodurch eine Umkehrung der vorgegebenen Bewegung des SMA-Elements bewirkt wird. Der bekannte Aktuator umfasst weiterhin eine Detektionseinheit, um ein Erreichen und Verlassen einer Endposition zu detektieren. Die Endposition wird in der gezeigten Ausführungsform durch Überbrücken eines Abschnitts des SMA-Elements und Messen eines durch die Überbrückung verringerten Widerstands erzielt.

Aus der DE 10 2015 113 029 A1 ist eine Steuereinrichtung für die Verstellung von Luftkissen bekannt. Zur Steuerung wird die Laufzeit einer Pumpe erfasst und unter Berücksichtigung der Fördermenge der Pumpe eine dem wenigstens einen Luftkissen zugeführte Luftmenge bzw. ein zugeführtes Luftvolumen ermittelt.

Aus der DE 10 2015 213 442 ist ein Verfahren zur Überwachung des Drucks in einer pneumatischen Sitzverstellungseinrichtung bekannt. Dazu wird der Druck in jeder Luftkammer oder einem Versorgungskanal mit einem Drucksensor gemessen.

Die bekannten Verfahren beruhen darauf, dass jeweils bei einer bestimmten Position des Stellelements eine Art Schalter betätigt wird, der durch einen Kippschalter oder eine Überbrückung realisiert ist. Solche mechanischen Schalter können durch Schmutzpartikel, Abrieb, Flüssigkeiten aber auch eine hohe Schaltfrequenz in ihrer Funktion beeinträchtigt werden.

Aus EP 0 940 742 A2 ist ein Massendurchflussregler bekannt, der dazu ausgebildet ist, den Durchfluss eines gasförmigen Fluids zu steuern. Der Massendurchflussregler weist ein Ventil und einem Sensor zum Messen des Fluiddurchflusses auf.

Aufgabe der Erfindung ist es, ein neues Sitzkomfortsystem, insbesondere eine gegenüber dem Stand der Technik verbesserte Sitzkomfortsystem anzugeben.

Diese Aufgabe wird durch ein Sitzkomfortsystem mit den Merkmalen des Anspruchs 1 gelöst. Zweckdienliche Ausgestaltungen ergeben sich aus den jeweiligen Unteransprüchen.

Das erfindungsgemäße Sitzkomfortsystem umfasst ein oder mehrere Luftkissen, wobei der Füllstand jedes Luftkissens über zumindest ein Ventil steuerbar ist, und ein oder mehrere Schaltungsanordnungen zur Ansteuerung des oder der Ventile. Insbesondere ist das Sitzkomfortsystem für den Einbau in einen Sitz, insbesondere einen Fahrzeugsitz, ausgebildet. Das Sitzkomfortsystem kann beispielsweise in einen Fahrzeugsitz eingesetzt werden oder eingesetzt sein. Bei dem Sitzkomfortsystem kann es sich beispielsweise um eine Lordosenstützvorrichtung und/oder Massageeinrichtung handeln. Das oder die Luftkissen sind üblicherweise in einem Sitz, insbesondere einem Fahrzeugsitz, angeordnet.

Das mindestens eine Ventil, zu dessen Ansteuerung die Schaltungsanordnung vorgesehen ist, umfasst zumindest einen Aktuator mit zumindest einem Stellelement, wobei das Stellelement zumindest zwischen einer ersten Stellung und einer zweiten Stellung verstellbar ist. Erste Stellung und zweite Stellung sind zwei voneinander verschiedene Stellungen, die als Öffnungs- und Schließstellung des Ventils oder als Zwischenstellungen gewählt werden können. Der Aktuator kann beispielsweise ein piezoelektrisches Element oder ein magnetisches Element, insbesondere ein elektromagnetisches Element, oder ein SMA-Element umfassen. Ein solches SMA-Element (Formgedächtnislegierungs-Element) ist insbesondere ein Draht oder Band aus einem SMA.

Die Schaltungsanordnung weist zumindest eine Treibereinheit zum Betätigen des Aktuators und eine Steuereinheit zum Steuern der Treibereinheit auf.

Die Schaltungsanordnung des erfindungsgemäßen Sitzkomfortsystems umfasst zumindest eine Luftmassenmessvorrichtung zur Messung einer das Ventil durchströmenden Luftmasse. Die durch das Ventil strömende Luftmasse kann beispielsweise, wie untenstehend näher erläutert wird, mittels eines Messdrahtes bestimmt werden, an dem die zu messende Luftmasse vorbeiströmt und hierbei beispielsweise eine Temperaturänderung an oder in der Umgebung des Messdrahtes hervorruft. Anhand der das Ventil durchströmenden Luftmasse kann somit bestimmt oder zumindest abgeschätzt werden, wieviel Luft bzw. welche Luftmenge sich in einem zugeordneten Luftkissen des Sitzkomfortsystems befindet. Beispielsweise können hierzu die Messwerte der Luftmassenmessvorrichtung mit hinterlegten Vergleichswerten verglichen und/oder parallel Referenzmessungen in Bereichen vorgenommen werden, an denen keine Luftströmung vorhanden ist, beispielsweise an zusätzlichen Referenzmessdrähten.

Weiter sieht die Erfindung vor, dass die Steuereinheit zum Steuern der Treibereinheit dazu geeignet ist, ein Ausgangssignal der Luftmassenmessvorrichtung zu verarbeiten. Die Steuereinheit ist somit derart eingerichtet, dass sie ein Ausgangssignal und damit ein Messergebnis der Luftmassenmessvorrichtung verarbeitet und dieses Messergebnis somit beispielsweise zum Steuern der Treibereinheit zum Betätigen des Aktuators verwendet, also beispielsweise beim Erreichen eines vorgegebenen Schwellenwertes der durchströmenden Luftmasse bzw. Luftmenge das Ventil schließt. Die erfindungsgemäße Schaltungsanordnung ermöglicht somit beispielsweise beim Füllen oder Entleeren von Luftkissen die, insbesondere kontaktlose, Erkennung eines oder mehrerer vorgegebener Füllzustände des Luftkissens und kann die Schaltungsanordnung entsprechend steuern oder regeln, beispielsweise das Ventil schließen oder öffnen.

Die Luftmassenmessvorrichtung erzeugt also, beispielsweise basierend auf der Messung von Temperatur und/oder Heizstrom und/oder elektrischer Leistungsaufnahme und/oder Widerstand mindestens eines Messdrahtes der Luftmassenmessvorrichtung und/oder der Veränderung dieser Messgrößen mit der Zeit, ein Ausgangssignal, das die durch das Ventil geströmte Luftmasse und damit auch die Luftmenge kennzeichnet. Dieses Ausgangssignal wird an die Steuereinheit weitergegeben, so dass die Steuereinheit beispielsweise die Treibereinheit basierend auf der gemessenen Luftmasse bzw. Luftmenge steuern oder regeln kann.

In Ausgestaltung umfasst die Luftmassenmessvorrichtung zumindest einen Messdraht und eine Messvorrichtung, wobei die Messvorrichtung
a. eine Widerstandsmessvorrichtung zur Messung des Widerstands des Messdrahtes und/oder
b. einen Temperatursensor und/oder eine Temperaturmessvorrichtung zur Messung einer Temperatur des Messdrahtes und/oder der Umgebungstemperatur des Messdrahtes und/oder
c. eine Strommessvorrichtung zur Messung eines Heizstroms des Messdrahtes und/oder
d. eine Leistungsmessvorrichtung zur Messung einer Eingangsleistung des Messdrahtes
umfasst. Die Änderung der Messwerte, die mittels der Messvorrichtung festgestellt wird, ist abhängig von der dem Messdraht entzogenen Wärmemenge durch die vorbeistreichende Luft und somit ein Indikator für die zu bestimmende Luftmasse. Im Sinne dieser Erfindung soll unter Widerstand der elektrische Widerstand verstanden werden. Das Ausgangssignal der jeweiligen Messvorrichtung wird an die Steuereinheit weitergegeben, sodass die Steuereinheit die Treibereinheit basierend auf dem gemessenen Wert der Messvorrichtung steuern oder regeln kann. Die Schaltungsanordnung kann dazu ausgelegt sein, dass entweder der Widerstand, der Strom und/oder die Leistung des Messdrahtes gemessen wird oder eine Bestromung erfolgt, somit also Messvorrichtung und Treibereinheit alternierend mit dem Messdraht wirkverbunden werden, was insbesondere dann zweckmäßig ist, wenn der Messdraht ein SMA-Element, also ein SMA-Draht ist, der gleichzeitig ein Element des Aktuators des Ventils ist. Dies wird nachfolgend näher erläutert. Im Falle einer Temperaturmessung können ebenfalls Messung und Ansteuerung der Treibereinheit gleichzeitig oder alternierend erfolgen. Es kann vorgesehen sein, dass die Steuereinheit auch die Widerstandsmessvorrichtung und/oder den Temperatursensor und/oder die Temperaturmessvorrichtung und/oder die Strommessvorrichtung und/oder die Leistungsmessvorrichtung steuert.

Die Steuereinheit kann in Ausgestaltung der Schaltungsanordnung des erfindungsgemäßen Sitzkomfortsystems über weitere Sensoren zur Strom-, Spannungs- und/oder Temperaturüberwachung verfügen. Die Steuereinheit kann in Ausgestaltung über Kommunikationsschnittstellen verfügen, um über in einem Fahrzeug vorhandene Schalter und/oder einen Bordcomputer gesteuert zu werden. Als weiteren Eingang kann die Steuereinheit über eine LIN (Local Interconnect Network)-Kommunikationsschnittstelle verfügen, welche insbesondere einen Sendeempfänger und/oder eine Schaltereingangsschnittstelle aufweist. Die Schaltereingangsschnittstelle ist insbesondere zum Verarbeiten widerstandsbasierter Schaltersignale geeignet, wobei die Schaltereingangsschnittstelle für eine Mehrzahl von Schaltereingängen ausgelegt sein kann, z.B. für die Sitzverstellung, insbesondere die Sitzposition, und für Lordose- und/oder Massagefunktionen. Die Steuereinheit kann einen Speicher zum Speichern von Daten umfassen.

In weiterer Ausgestaltung der Schaltungsanordnung des erfindungsgemäßen Sitzkomfortsystems sind Luftmassenmessvorrichtung und/oder Steuereinheit dazu eingerichtet, die Luftmasse aus
a. der Temperatur des Messdrahtes und dem Heizstrom und/oder
b. der Temperatur des Messdrahtes und der Eingangsleistung und/oder
c. dem Widerstand des Messdrahtes und dem Heizstrom und/oder
d. dem Widerstand des Messdrahtes und der Eingangsleistung zu ermitteln.

Der Aktuator kann ein SMA-Element umfassen. In einer Ausgestaltung kann in diesem Fall vorgesehen sein, dass das SMA-Element ein SMA-Draht ist und dass die Luftmassenmessvorrichtung zumindest einen Messdraht umfasst, wobei der SMA-Draht dieser Messdraht oder einer der Messdrähte ist. Unter SMA-Draht wird hierbei ein draht- oder bandförmiges SMA-Element verstanden. Gegebenenfalls können ein oder mehrere weitere Messdrähte der Luftmassenmessvorrichtung vorgesehen sein. Eine Weiterbildung dieser Ausgestaltung sieht vor, dass die Luftmassenmessvorrichtung eine Messvorrichtung, insbesondere eine Widerstandsmessvorrichtung, umfasst und dass die Schaltungsanordnung dazu ausgelegt ist, dass die Messvorrichtung und die Treibereinheit alternierend mit dem SMA-Element, konkret dem SMA-Draht, wirkverbunden sind. Diese alternierende Ansteuerung kann durch die entsprechend eingerichtete Steuereinheit verwirklicht sein. Es wird somit entweder die Luftmasse mit dem SMA-Draht in seiner Funktion als Messdraht gemessen oder es erfolgt eine Bestromung des SMA-Drahts in seiner Funktion als Aktuator. Diese beiden Funktionen werden alternierend durchgeführt, das heißt Luftmassenmessvorrichtung und Treibereinheit werden alternierend mit dem SMA-Draht wirkverbunden. Dazu kann insbesondere die Steuereinheit neben der Treibereinheit auch die Luftmassenmessvorrichtung steuern.

In einer Ausführungsvariante der Ausgestaltung, bei der der Aktuator ein SMA-Element, beispielsweise einen SMA-Draht, umfasst, kann vorgesehen sein, dass die Luftmassenmessvorrichtung zumindest einen Messdraht umfasst, wobei dieser Messdraht oder zumindest einer dieser Messdrähte ein zusätzlich zum SMA-Element vorgesehener, separater Draht ist. Gegebenenfalls können ein oder mehrere weitere Messdrähte der Luftmassenmessvorrichtung vorgesehen sein, wobei auch das SMA-Element ein SMA-Draht sein kann, der als weiterer Messdraht vorgesehen ist.

Der separate Draht ist somit getrennt vom SMA-Element des Aktuators angeordnet. Bei diesem separaten Draht kann es sich um einen Draht aus einem SMA-Material oder aus einem anderen metallischen Material, z.B. Wolfram oder Platin, oder aus einer Legierung handeln. Der separate Draht kann beispielsweise parallel zu dem oder den SMA-Elementen geschaltet sein, aber davon verschieden, also getrennt von diesen SMA-Elementen, angesteuert werden.

Die Schaltungsanordnung, insbesondere die Messvorrichtung, kann eine Auswerteeinheit umfassen oder mit einer Auswerteeinheit verbunden sein. Die Auswerteeinheit kann beispielsweise konkrete Messwerte der Luftmassenmessvorrichtung, beispielsweise Widerstandsmesswerte, Temperaturwerte, Stromstärken und/oder Leistungswerte, aufnehmen und auswerten und hieraus ein Ausgangssignal erzeugen, das an die Steuereinheit weitergebenen wird. Dieses Ausgangssignal kann insbesondere ein Signal zur Übermittlung des Erreichens einer Endbedingung umfassen.

In einer Ausgestaltung umfasst die Schaltungsanordnung einen ASIC (ASIC: application-specific integrated circuit - anwendungsspezifische integrierte Schaltung), welcher eine oder mehrere der folgenden Komponenten aufweist: die Treibereinheit, insbesondere mit einem oder mehreren SMA-Treibern, eine bzw. die vorstehend genannte Messvorrichtung der Luftmassenmessvorrichtung, insbesondere mit einer Auswerteeinheit und/oder einem Speicher, und die Steuereinheit. Durch die Ausgestaltung mit einem ASIC wird die Schaltungsanordnung kleiner und kann kostengünstiger hergestellt werden. Dabei kann die Messvorrichtung, wie vorstehend ausgeführt, eine Tempermessvorrichtung, Widerstandsmessvorrichtung, Strommessvorrichtung und/oder Leistungsmessvorrichtung sein.

In einer weiteren Ausgestaltung umfasst die Schaltungsanordnung weitere Sensoren, insbesondere einen Endpositionsschalter. Beispielsweise kann ein solcher Endpositionsschalter sowohl als Sicherheitsmaßnahme als auch als Kalibrierhilfe dienen.

In Ausgestaltung umfasst die Schaltungsanordnung eine Vielzahl von Aktuatoren, wobei jedem Aktuator eine Treibereinheit, insbesondere eine Treibereinheit mit einem SMA-Treiber, zur Betätigung des jeweiligen Aktuators oder für jeden Aktuator ein SMA-Treiber in einer Treibereinheit zugeordnet ist. Zweckmäßigerweise umfasst die Schaltungsanordnung einen Multiplexer, welcher mit jedem der SMA-Elemente des Aktuators so verbunden ist, dass der Widerstand jedes SMA-Elements einzeln gemessen werden kann. Somit wird mittels des Multiplexers jedes SMA-Element nacheinander mit der Widerstandsmessvorrichtung zum Messen des Widerstands verbunden.

In weiterer Ausgestaltung umfasst die Schaltungsanordnung, beispielsweise die Auswerteeinheit und/oder die Steuereinheit, einen Speicher zum Speichern von Daten. Bei dem Speicher kann es sich alternativ auch um einen gemeinsamen Speicher der Auswerteeinheit und der Steuereinheit handeln.

Es kann vorgesehen sein, dass die Auswerteeinheit und/oder die Steuereinheit einen Verlauf nacheinander gemessener Werte, insbesondere Temperaturmesswerte, Strommesswerte, Widerstandswerte oder (Eingangs-)leistungswerte, desselben SMA-Elementes auswertet und/oder Messwerte mit einem oder mehreren vorgegebenen Werten vergleicht.

Die Steuereinheit ist beispielsweise zur Steuerung mittels Pulsweitenmodulation ausgelegt. Dazu umfasst die Steuereinheit beispielsweise eine Pulsweitenmodulationseinheit. Somit werden die SMA-Elemente über die zugeordneten SMA-Treiber der Treibereinheit nacheinander innerhalb eines Duty-Cycles für eine vorgegebene Pulsweite und Pulshöhe bestromt und somit geheizt. Die Pulsweitenmodulationseinheit kann in Ausgestaltung ein Zeitsignal an die Messvorrichtung, beispielsweise eine Widerstandsmessvorrichtung, und/oder die Luftmassenmessvorrichtung ausgeben.

Es kann ferner vorgesehen sein, dass die Schaltungsanordnung, insbesondere die Messvorrichtung und/oder die Auswerteeinheit, einen Signalverstärker und/oder einen Rauschunterdrücker umfassen.

Die Messvorrichtung, insbesondere die Widerstandsmessvorrichtung, umfasst in einer Ausgestaltung zwei oder mehr Multiplexer, welche jeweils mit einem Teil der SMA-Elemente verbunden sind. Diese Ausgestaltung ist für große Systeme mit einer Vielzahl von SMA-Elemente möglich. Beispielsweise können pro Multiplexer 20 SMA-Elemente vorgesehen sein.

Zweckmäßigerweise ist jeweils zwischen Messvorrichtung, insbesondere Widerstandsmessvorrichtung, und SMA-Element jeweils ein Vorwiderstand seriell geschaltet. Dieser Vorwiderstand dient in Wesentlichen zur Reduktion von Stromspitzen und reduziert somit die Gefahr einer Überlastung der SMA-Elemente.

Es kann auch vorgesehen sein, dass die Schaltungsanordnung weitere Sensoren als die vorstehend bereits beschriebenen umfasst, beispielsweise einen Endpositionsschalter.

Ein Verfahren zur Ansteuerung des oder der Ventile des erfindungsgemäßen Sitzkomfortsystems mittels der Schaltungsanordnung des Sitzkomfortsystems umfasst die Schritte:
a. Messen einer Luftmasse,
b. Nachfolgend Ansteuern des Aktuators, insbesondere eines SMA-Elementes des Aktuators, mittels der Treibereinheit in Abhängigkeit der gemessenen Luftmasse,
c. Zyklisches Widerholen des Messens und Ansteuerns bis zum Erreichen einer Endbedingung.

Die Endbedingung kann beispielsweise das Erreichen eines vorgegebenen Luftmassenwertes und/oder einer vorgegebenen Änderung und/oder eines Füllstands, beispielsweise eines Luftkissens, sein oder umfassen.

Bei der Erhitzung eines SMA-Elementes, insbesondere indem das SMA-Element von einem Heizstrom durchflossen wird, ändert sich dessen Widerstand. Eine solche Widerstandsänderung ist beispielweise von Song "Resistance modelling of SMA Wire actuators", International Workshop Smart Materials Structures & NDT in Aerospace, NDT in Canada 2011 veröffentlicht worden. Somit kann einer vorgegebenen Längenänderung, insbesondere einer Verkürzung, eine Änderung eines Widerstands zugeordnet werden. Die Änderung des Widerstands ist allerdings nicht linear, umfasst aber lineare und nahezu lineare Bereiche. In Versuchen hat sich herausgestellt, dass eine Steigung der Widerstandskurve beim Erreichen eines Endpunktes des Aktuators eine Vorzeichenumkehr hat, so dass die Widerstandskurve einen Umkehrpunkt, d.h. einen Nulldurchgang der zweiten Ableitung der Widerstandskurve, aufweist. Die Bestimmung des Umkehrpunktes kann über den Vergleich mit dem oder den vorher gemessenen Widerstandswerten erfolgen. Eine Kombination aus Detektion des Umkehrpunktes und Vergleich mit einem vorgegebenen Absolutwert erhöht die Genauigkeit des Verfahrens. Wird zur Bestimmung der Luftmasse ein Draht mit einem anderen Material, z.B. einem Metall, eingesetzt, weisen Widerstand und Temperatur einen anderen Zusammenhang auf.

Die Endbedingung kann insbesondere für einen Normalbetrieb so gewählt werden, dass das Stellelement in eine Zwischenstellung nahe der Öffnungs- oder Schließstellung gebracht wird, so dass ein Endpunkt des Aktuators mit dem erfindungsgemäßen Verfahren nicht erreicht wird. Dies reduziert die mechanische Belastung der Ventile.

Zweckmäßigerweise umfasst das Verfahren eine Ansteuerung mittels Pulsweitenmodulation.

In Ausgestaltung umfasst das Verfahren zusätzlich mindestens einen der folgenden Schritte, wobei die Luftmassenmessvorrichtung zumindest einen Messdraht umfasst:
a. Messen eines Widerstands des Messdrahtes und/oder
b. Messen einer Temperatur und/oder einer Umgebungstemperatur des Messdrahtes und/oder
c. Messen eines Heizstroms des Messdrahtes und/oder
d. Messen einer Eingangsleistung des Messdrahtes.

Auch kann das Verfahren das Messen des Widerstands aller SMA-Elemente umfassen, wobei die Messung des Widerstands aller SMA-Elemente in einer gemeinsamen Ansteuerungstotzeit erfolgt oder wobei die Messung eines SMA-Elementes jeweils zwischen der Ansteuerung eines SMA-Elementes und der Ansteuerung eines anderen, insbesondere des nachfolgenden SMA-Elementes erfolgt. In diesem Fall sind der Schaltungsanordnung zwei oder mehrere Ventile zugeordnet und entsprechend zwei oder mehrere Aktuatoren vorgesehen, die jeweils ein SMA-Element umfassen.

In einer weiteren Ausgestaltung wird eine notwendige Leistung zur Betätigung des Aktuators mittels der Treibereinheit berechnet oder aus einer Tabelle abgelesen.

Es kann vorgesehen sein, dass die Messwerte der Luftmassenmessvorrichtung und/oder der Messvorrichtung herangezogen werden und anhand hinterlegter Vergleichswerte und/oder einer hinterlegten Wertetabelle aus diesen Messwerten die Luftmasse und/oder die Luftmenge und/oder ein Ansteuersignal für die Treibereinheit ermittelt und/oder ausgegeben wird. Dies kann beispielsweise in der Auswerteeinheit und/oder in der Steuereinheit erfolgen. Auch können alternativ oder zusätzlich die Messwerte beispielsweise mit Vergleichswerten einer parallel durchgeführten Referenzmessung, beispielsweise mittels eines Referenzmessdrahtes, vergleichen werden und hieraus die Luftmasse und/oder die Luftmenge und/oder Ansteuersignale für die Treibereinheit ermittelt und/oder ausgegeben werden.

Das oder die Ventile des erfindungsgemäßen Sitzkomfortsystems können in einer Ausgestaltung ein Ventilgehäuse umfassen, wobei das Ventilgehäuse beispielsweise einen Gehäusedeckel, einen Gehäuseboden und ein zwischen dem Gehäusedeckel und dem Gehäuseboden angeordnetes Zwischengehäuse aufweisen kann. Das Ventilgehäuse weist zumindest eine erste Öffnung und zumindest eine zweite Öffnung auf und das Ventilgehäuse umschließt einen Ventilraum. Dieser kann eine Strömungskammer und eine Betätigungskammer umfassen. Das Ventil umfasst einen Aktuator mit einem Stellelement zum Öffnen und Schließen des Ventils, beispielsweise der ersten und/oder zweiten Öffnung oder einer Öffnung innerhalb des Ventils, beispielsweise einer Öffnung zwischen der Strömungs- und der Betätigungskammer, sowie zweckmäßigerweise ein Rückstellelement. Der Aktuator kann ferner ein SMA-Element, insbesondere einen SMA-Draht, umfassen. Alternativ kann der Aktuator auch ein piezoelektrisches Element oder ein magnetisches Element umfassen.

Das Ventilgehäuse kann zumindest eine von der Strömungskammer in die Betätigungskammer mündende Ventilöffnung aufweisen, wobei innerhalb der Betätigungskammer das zwischen einer Schließstellung zum Verschließen der Ventilöffnung und einer Öffnungsstellung zur Freigabe der Ventilöffnung axial bewegbare Stellelement, ein zur Betätigung des Stellelementes in Öffnungsrichtung dienendes draht- oder bandförmiges SMA-Element aus einer Formgedächtnislegierung, ein zur Bewegung des Stellelementes in Schließrichtung dienendes Rückstellelement und eine Leiterplatte angeordnet sind, wobei das SMA-Element mit einem Abschnitt an dem Stellelement fixiert und zur Beaufschlagung mit Strom mit zumindest einem Ende mit der Leiterplatte elektrisch verbunden ist.

In zweckmäßiger Ausgestaltung ist das SMA-Element mit einem mittleren Abschnitt an dem Stellelement fixiert und mit beiden Enden mit der Leiterplatte verbunden.

Ein Messdraht der Schaltungsanordnung ist zweckmäßigerweise im Ventilraum des Ventils angeordnet.

Das erfindungsgemäße Sitzkomfortsystem weist mehrere Ventile, wie vorstehend beschriebenen, auf, die eine Ventilanordnung bilden. Dabei ist die Schaltungsanordnung zur Steuerung jedes Ventils in eine gemeinsame Schaltungsanordnung integriert. Es kann vorgesehen sein, dass alle Ventilgehäuse der Ventile zusammen einstückig ausgebildet sind. Insbesondere die den Ventilraum eines Ventils jeweils umschließenden Ventilgehäuse, insbesondere umfassend Zwischengehäuse und/oder Gehäusedeckel und/oder Gehäuseböden, können einstückig ausgebildet sein.

In Ausgestaltung weist zumindest ein erster Teil der mehreren Ventile einen gemeinsamen Druckanschluss auf, der jeweils in den Ventilraum, insbesondere in die Strömungskammer, besonders bevorzugt in einen ersten Bereich der Strömungskammern oder einen die ersten Bereiche der Strömungskammern umfassenden Bereich mündet oder jeweils mit dem Ventilraum, insbesondere der Strömungskammer, besonders bevorzugt dem ersten Bereich der Strömungskammer oder dem Bereich über zumindest einen Luftkanal verbunden ist. Zumindest ein zweiter Teil der mehreren Ventile kann eine gemeinsame Öffnung zum Verbinden mit der Atmosphäre aufweisen, die insbesondere in den ersten Bereich der Strömungskammer oder einen die ersten Bereiche der Strömungskammern umfassenden Bereich mündet oder mit den ersten Bereichen der Strömungskammern verbunden ist. Der Messdraht kann, insbesondere als SMA-Draht, in jedem Ventil aufgenommen sein.

Alternativ zur vorstehend beschriebenen Ausgestaltung kann der Messdraht auch nicht in jedem Ventil der Ventilanordnung aufgenommen sein, sondern der Draht der Luftmassenmessvorrichtung kann in dem Luftkanal zum Druckanschluss und/oder zu der gemeinsamen Öffnung zum Verbinden mit der Atmosphäre angeordnet sein. Die Luftmassenmessvorrichtung kann somit jeweils einen Messdraht für jedes Ventil oder einen Messdraht zusammen für einen ersten Teil der mehreren Ventile und/oder einen Messdraht zusammen für einen zweiten Teil der mehreren Ventile umfassen.

In weiterer Ausgestaltung ist die Luftmassenmessvorrichtung derart in die Schaltungsanordnung integriert, dass die Luftmassenmessung nur durchgeführt wird, wenn genau ein Ventil geöffnet ist.

Das erfindungsgemäße Sitzkomfortsystem umfasst ein oder mehrere erfindungsgemäße Ventile und/oder eine erfindungsgemäße Ventilanordnung und/oder eine oder mehrere erfindungsgemäße Schaltungsanordnungen und zusätzlich ein oder mehrere Luftkissen, wobei der Füllstand jedes Luftkissens über zumindest ein Ventil steuerbar ist. Insbesondere ist das Sitzkomfortsystem für den Einbau in einen Sitz, insbesondere einen Fahrzeugsitz, ausgebildet.

Die Erfindung wird nachstehend auch hinsichtlich weiterer Merkmale und Vorteile anhand der Beschreibung von Ausführungsbeispielen und unter Bezugnahme auf die beiliegenden Zeichnungen näher erläutert. Es zeigen jeweils in einer schematischen Pri nzi pskizze:
- Fig. 1A: eine Schaltungsanordnung nach dem Stand der Technik,
- Fig. 1B: ein SMA-Ventil mit einem Aktuator nach dem Stand der Technik,
- Fig. 1C: eine zu Fig. 1B alternative Ausgestaltung eines Aktuators eines Ventils nach dem Stand der Technik,
- Fig. 2: eine erste Ausgestaltung einer Schaltungsanordnung eines erfindungsgemäßen Sitzkomfortsystems,
- Fig. 3: eine Ausgestaltung eines Ventils eines erfindungsgemäßen Sitzkomfortsystems umfassend einen Aktuator mit einem SMA-Element,
- Fig. 4: eine zweite Ausgestaltung einer Schaltungsanordnung eines erfindungsgemäßen Sitzkomfortsystems,
- Fig. 5: eine dritte Ausgestaltung einer Schaltungsanordnung eines erfindungsgemäßen Sitzkomfortsystems,
- Fig. 6: ein Beispiel für einen ASIC für eine Schaltungsanordnung eines erfindungsgemäßen Sitzkomfortsystems,
- Fig. 7: eine Ausgestaltung einer Ventilanordnung für ein erfindungsgemäßes Sitzkomfortsystem mit mehreren Ventilen,
- Fig. 8: eine Ausgestaltung eines erfindungsgemäßen Sitzkomfortsystems.

Fig. 1A, Fig. 1B und Fig. 1C zeigen den Stand der Technik und wurden bereits eingangs beschrieben.

Fig. 2 zeigt eine erste Ausgestaltung einer Schaltungsanordnung 1. Die Schaltungsanordnung 1 ist zusammen mit mehreren SMA-Elementen 100-1 bis 100-N, insbesondere SMA-Drähten, welche insbesondere wie in den Fig. 1B und Fig. 1C dargestellt jeweils Teil eines Aktuators 103 und somit eines Ventils 120 sind, zum Ansteuern mehrerer Ventile 120 eines Sitzkomfortsystem 2 ausgelegt.

Die Schaltungsanordnung 1 umfasst eine Messvorrichtung 5 und eine Steuereinheit 30. Die Messvorrichtung 5 ist mit jedem der SMA-Elemente 100-1 bis 100-N verbindbar oder verbunden. Die Messvorrichtung 5 bildet mit jedem der SMA-Elemente 100-1 bis 100-N eine Luftmassenmessvorrichtung 305-1 bis 305-N. Die Messvorrichtung 5 kann als Widerstandsmessvorrichtung, Temperaturmessvorrichtung, Strommessvorrichtung und/oder Leistungsmessvorrichtung ausgestaltet sein. Die SMA-Elemente 100-1 bis 100-N sind mit einer durch den Pfeil dargestellten Spannungsquelle U verbunden. Die Steuereinheit 30 ist über SMA-Treiber 20 mit jedem der SMA-Elemente 100-1 bis 100-N verbindbar oder dauerhaft verbunden. Die SMA-Treiber 20 sind in einer Treibereinheit 6 angeordnet. Die Messvorrichtung 5 erzeugt ein Ausgangssignal, das kabelgebunden oder drahtlos an die Steuereinheit 30 übermittelt wird und dort als Eingangssignal für die Steuerung oder Regelung der SMA-Treiber 20 genutzt wird.

Die Messvorrichtung 5 umfasst optional einen Multiplexer 12, welcher mit jedem der SMA-Elemente 100-1 bis 100-N verbindbar oder verbunden ist, so dass insbesondere durch Anlegen eines Messstroms mittels einer Stromquelle 13 ein Widerstand eines der SMA-Elemente 100-1 bis 100-N gemessen wird. Weiterhin ist zweckmäßigerweise ein Signalverstärker 14 in der Messvorrichtung 5 vorgesehen, welcher eine Offset-Korrektur aufweisen kann. Das erhaltene Messsignal kann nun in einer Auswerteeinheit 8 zur Bestimmung der Luftmasse ausgewertet werden. Im gezeigten Beispiel ist die Auswerteeinheit 8 in der Messvorrichtung 5 angeordnet. Alternativ kann sie als separates Bauteil zwischen Messvorrichtung 5 und Steuereinheit 30 angeordnet sein.

Die Steuereinheit 30 der in Fig. 2 gezeigten Schaltungsanordnung 1 kann zum Betätigen der SMA-Elemente 100-1 bis 100-N mittels Pulsweitenmodulation ausgelegt sein.

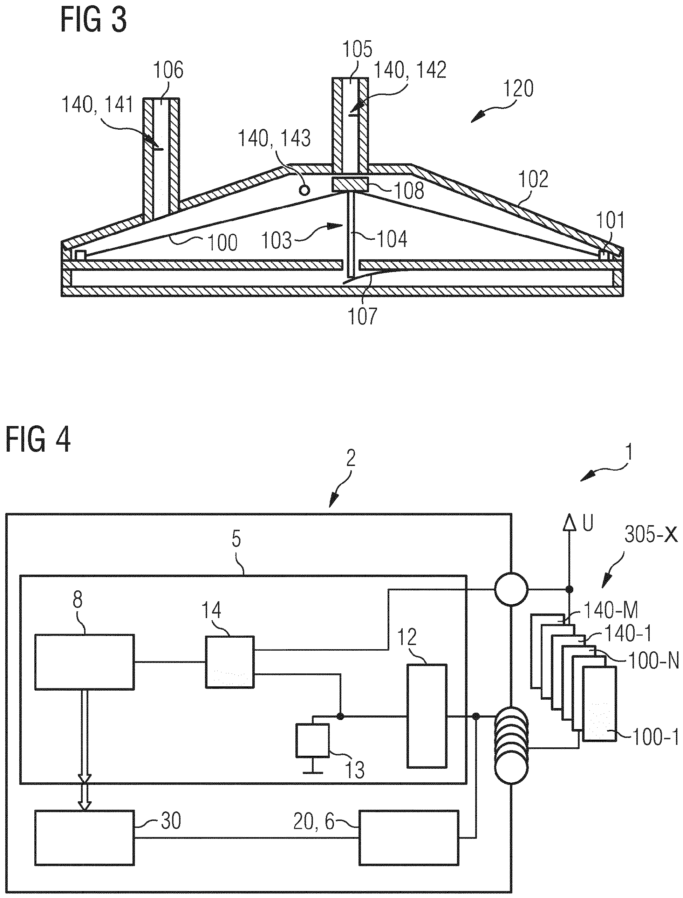

Fig. 3 zeigt eine alternative Ausgestaltung eines Ventils 120. In diesem Ventil 120 ist, im Unterschied zum Ventil 120 gemäß Fig. 1B, ein zu dem SMA-Element 100 separater Draht 140 als Messdraht an verschiedenen Positionen im Ventil 120 angeordnet. Fig. 3 zeigt als Beispiele drei verschiedene Position, an denen der Draht 140 angeordnet sein kann. Beispielsweise kann der Draht 140 als Draht 141 in der zweiten Öffnung 106 und/oder als Draht 142 in der ersten Öffnung 105 des Ventils 120 angeordnet sein. Alternativ oder zusätzlich kann der Draht 140 in einer Ventilkammer, beispielsweise einer Strömungskammer des Ventils als Draht 143 angeordnet sein. Bei dieser in Fig. 3 gezeigten Ausgestaltung des Ventils 120 kann alternativ oder zusätzlich auch der Draht 140, insbesondere als Draht 141 und/oder Draht 142 und/oder Draht 143, mit der Messvorrichtung 5 (siehe beispielsweise Fig. 4) verbindbar oder verbunden sein und zusammen mit der Messvorrichtung 5 jeweils eine Luftmassenmessvorrichtung bilden.

Fig. 4 zeigt eine zweite Ausgestaltung der Schaltungsanordnung 1, bei welcher in den jeweiligen Ventilen 120 zusätzlich separate Drähte 140-1 bis 140-M angeordnet sind, wie beispielsweise in Fig. 3 gezeigt, die ebenso wie die SMA-Elemente 100-1 bis 100-N jeweils mit der Messvorrichtung 5 eine Luftmassenmessvorrichtung 305-X bilden, so dass die Luftmassenmessung auch an den Drähten 140-1 bis 140-M durchgeführt werden kann und die Ansteuerung des jeweiligen SMA-Elements 100-1 bis 100-N des jeweiligen Ventils 120 über den SMA-Treiber 20 anhand dieser Messung erfolgt. Die separaten Drähte 140-1 bis 140-M sind in der in Fig. 4 gezeigten Ausführungsform parallel zu den SMA-Elementen 100-1 bis 100-N geschaltet, allerdings können die separaten Drähte 140-1 bis 140-M für die Messung jeweils gesondert für die jeweilige Messung angesteuert werden. Alternativ oder zusätzlich zu dieser Ansteuerung kann aber auch an den SMA-Elementen 100-1 bis 100-N beispielsweise ein Widerstand mittels der Schaltungsanordnung 1 gemessen werden. Die Anzahl M der separaten Drähte 140-1 bis 140-M und die Anzahl N der SMA-Elemente 100-1 bis 100-N kann gleich oder verschieden sein. Wenn der Aktuator 103 der Ventile 120 alternativ zu einem SMA-Element das Stellelement 104 mit einem piezoelektrischen Element oder einem magnetischen, insbesondere elektromagnetischen Element ansteuert, kann dieses piezoelektrische oder magnetische Element analog zu den in Fig. 4 gezeigten SMA-Elementen 100-1 bis 100-N auf Basis der jeweiligen Messwerte der Luftmassenmessung mit dem jeweiligen Draht 140-1 bis 140-M angesteuert werden.

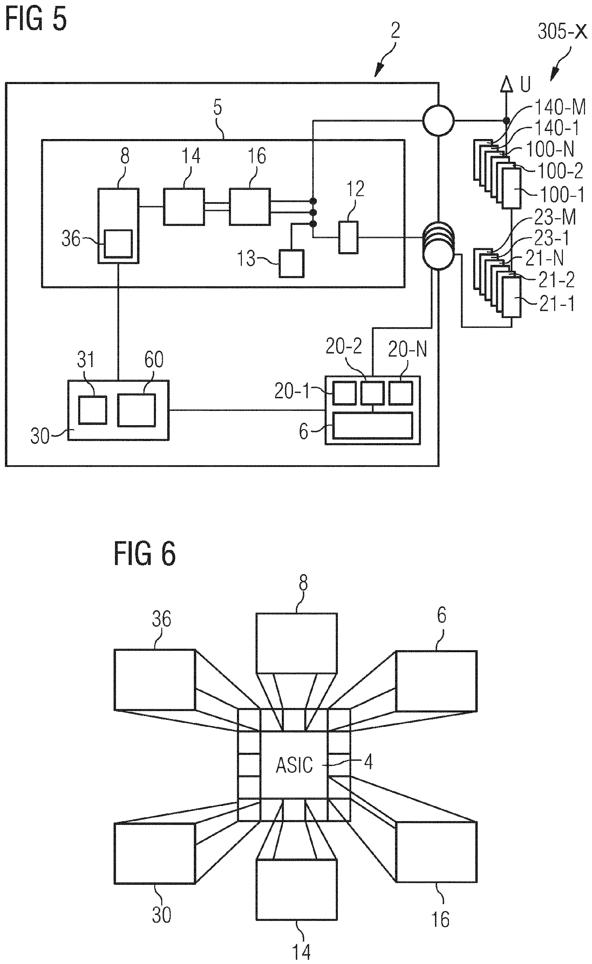

Fig. 5 zeigt eine dritte Ausgestaltung einer Schaltungsanordnung 1. Die Schaltungsanordnung 1 unterscheidet sich von der in Fig. 2 dargestellten Schaltungsanordnung 1 durch die Vorwiderstände 21-1 bis 21-N, die jeweils vor die SMA-Elemente 100-1 bis 100-N geschaltet sind. Optional sind auch hier, wie in Fig. 4 dargestellt, zusätzlich zu den SMA-Elementen 100-1 bis 100-N separate Drähte 140-1 bis 140-M angeordnet, wie in Fig. 5 dargestellt, vor die ebenfalls jeweils ein Vorwiderstand 23-1 bis 23-M geschaltet ist. Die Vorwiderstände 21-1 bis 21-N und die Vorwiderstände 23-1 bis 23-M ergänzen somit jeweils die jeweilige Luftmassenmessvorrichtung 305-X.

Weiterhin unterscheidet sich die Schaltungsanordnung 1 gemäß Fig. 5 von der Schaltungsanordnung 1 nach Fig. 2 darin, dass in der Messvorrichtung 5 ein Filter 16 zusätzlich zum Verstärker 14 angeordnet ist. Dabei können grundsätzlich auch mehrerer Filter- und Verstärkerstufen und/oder Integrationselemente, die der Signalverbesserung dienen, zum Einsatz kommen.

Die Auswerteeinheit 8 umfasst in der gezeigten Ausgestaltung gemäß Fig. 5 einen Speicher 36. Ein externer Speicher, auf den die Auswerteeinheit 8 zugreifen kann, ist alternativ oder zusätzlich auch möglich.

Die Steuereinheit 30 umfasst neben dem in Fig. 5 dargestellten Eingang 31, der auch in den anderen Ausgestaltungen vorhanden sein kann und beispielsweise zur Eingabe eines Steuersignals, welches drahtlos oder kabelgebunden übermittelt werden kann, ausgebildet sein kann, eine Pulsweitenmodulationsvorrichtung 60, die mit der Treibereinheit 6 und damit den SMA-Treibern 20-1 bis 20-N verbunden ist. Optional kann die Steuereinheit 30 zur Steuerung der Messvorrichtung 5 ausgelegt sein.

Wie bereits anhand von Fig. 4 beschrieben, können auch hier separate Drähte 140-1 bis 140-M in einfacher Weise in die Schaltungsanordnung 1 integriert werden.

Fig. 6 zeigt einen ASIC 4, der zur Verwirklichung der Schaltungsanordnung 1 eingesetzt werden kann. Dieser ASIC 4 kann die Komponenten der Messvorrichtung 5, beispielsweise die Auswerteeinheit 8 und/oder den Speicher 36 und/oder den Verstärker 14 und/oder den Filter 16, umfassen. Weiter kann der ASIC 4 die Steuereinheit 30, beispielsweise mit der Pulsweitenmodulationsvorrichtung 60, umfassen. Weiterhin kann, wenn der ASIC 4 die Steuereinheit 30 umfasst, der ASIC insbesondere auch den Eingang 31, ausgelegt beispielsweise für die Eingabe eines Steuersignals, umfassen, welches drahtlos oder kabelgebunden übermittelt werden kann. Optional kann der ASIC 4 auch die Treibereinheit 6 mit den SMA-Treibern 20 umfassen.

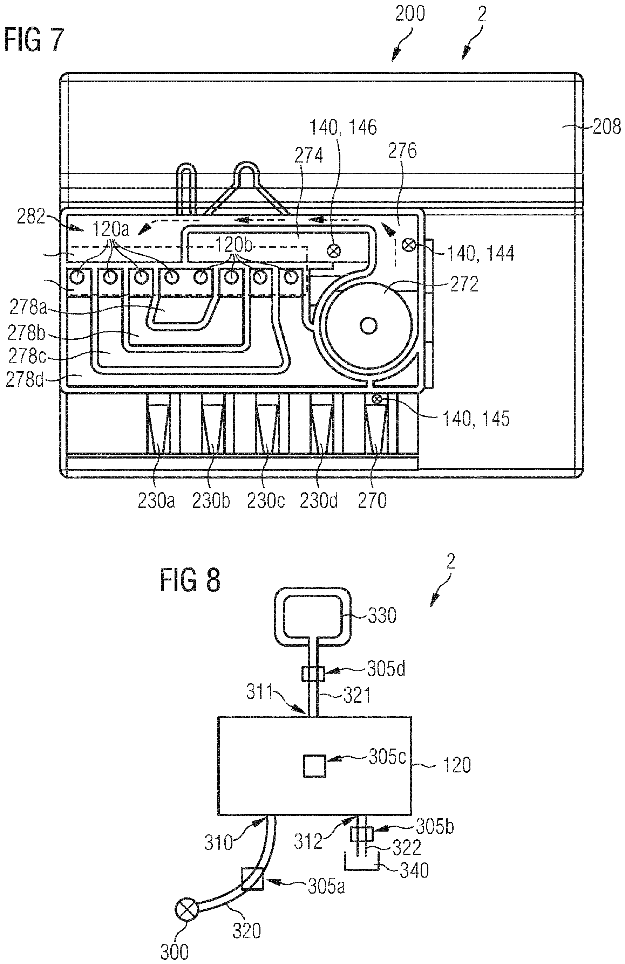

In Fig. 7 zeigt eine Ventilanordnung 200 eines Sitzkomfortsystems 2 mit mehreren Ventilen, konkret ersten Ventilen 120a und zweiten Ventilen 120b, die mit einer Schaltungsanordnung 1 angesteuert werden. Die Ansicht zeigt einen Ausschnitt eines Zwischengehäuses 208 des Sitzkomfortsystems 2. Über einen gemeinsamen Druckanschluss 270, der mit einer pneumatischen Pumpe verbindbar ist, wird einem ersten Strömungsbereich 282, der den ersten Ventilen 120a zugeordnet ist, vorliegend vier Ventilen 120a, über einen von dem Zwischengehäuses 208 ausgebildeten Luftkanal 276 Luft zugeführt (angedeutet durch gestrichelte Pfeile). Zwischen dem Druckanschluss 270 und dem ersten Strömungsbereich 282 ist innerhalb des Luftkanales 276 ein Rückschlagventil 272 angeordnet. Zweite Ventile 120b, vorliegend vier zweite Ventile 120b, sind über einen zweiten Strömungsbereich 274 an eine nicht dargestellte gemeinsame Öffnung zur Atmosphäre angebunden. An den dem ersten Strömungsbereich 282 bzw. dem zweiten Strömungsbereich 274 strömungstechnisch gegenüberliegenden Seiten der Ventile 120a, 120b sind jeweils ein Ventil 120a und ein Ventil 120b über einen Luftkanal 278a, 278b, 278c, 278d miteinander verbunden.

An Verbraucheranschlüsse 230a, 230b, 230c, 230d können Luftkissen angeschlossen sein. Wird ein Luftkissen entlüftet, strömt die darin enthaltene Luft zunächst über den jeweiligen Verbraucheranschluss 230a, 230b, 230c, 230d in den jeweils zugeordneten Luftkanal 278a, 278b, 278c, 278d und dann über das zugeordnete geöffneten zweite Ventil 120b in den zweiten Strömungsbereich 274 und von dort zur Atmosphäre. Das korrespondierende erste Ventil 120a, das an denselben Luftkanal 278a, 278b, 278c, 278d angeschlossen ist, ist in diesem Fall geschlossen. Wird ein Luftkissen mit Druckluft gefüllt, strömt Luft vom Druckanschluss 270 über den ersten Strömungsbereich 282 und das geöffnete zugeordnete erste Ventil 120a und den jeweiligen Luftkanal 278a, 278b, 278c, 278d und den jeweiligen Verbraucheranschluss 230a, 230b, 230c, 230d in das Luftkissen. Das korrespondierende zweite Ventil 120b ist in diesem Fall geschlossen.

Mit anderen Worten: Jeweils ein erstes Ventil 120a und ein zweites Ventil 120b sind einem Luftkissen bzw. allgemein einem Luftspeicher zugeordnet und mit diesem verbunden, wobei das erste Ventil 120a zur Befüllung des Luftkissens mit Luft und das zweite Ventil 120b zum Entleeren des Luftkissens dient.

In einem solchen System, das in Fig. 7 nicht limitierend dargestellt ist, können zum einen die SMA-Elemente, insbesondere SMA-Drähte, der Ventile 120a, 120b als Messdraht der Luftmassenmessvorrichtung und zum anderen separate Drähte 140 als Messdraht der Luftmassenmessvorrichtung eingesetzt werden. Hinsichtlich der separaten Drähte 140 ist die Anordnung in einem dem jeweiligen Ventil 120a, 120b ausschließlich zugeordneten Bereich, wie in Fig. 3 beispielhaft dargestellt, möglich. Alternativ oder zusätzlich kann ein Draht 140 auch in einem gemeinsamen Luftkanal 276, in Fig. 7 zusätzlich durch das Bezugszeichen 144 bezeichnet, und/oder im gemeinsamen Druckanschluss 270, in Fig. 7 zusätzlich durch das Bezugszeichen 145 bezeichnet, und/oder im zweiten Strömungsbereich 274, in Fig. 7 zusätzlich durch das Bezugszeichen 146 markiert, angeordnet sein.

Fig. 8 zeigt ein Sitzkomfortsystem 2. Das Sitzkomfortsystem 2 umfasst in der gezeigten Darstellung ein Ventil 120, welches eine erste Ventilöffnung 310, eine zweite Ventilöffnung 311 und eine dritte Ventilöffnung 312 aufweist. Die erste Ventilöffnung 310 ist mit einer Pumpe 300 über eine Fluidleitung 320 verbunden. Die zweite Ventilöffnung 311 ist über eine Fluidleitung 321 mit einem Luftkissen 330 verbunden. Die dritte Ventilöffnung 312 ist mit einer Atmosphärenöffnung 340 über eine weitere Fluidleitung 322 verbunden. Komponenten einer oder mehrerer Luftmassenmessvorrichtungen, insbesondere die Messdrähte der Luftmassenmessvorrichtungen, können in einer oder mehreren der Fluidleitungen 320, 321, 322 angeordnet sein. Symbolisch ist in Fig. 8 die der Fluidleitung 320 zugeordnete Luftmassenmessvorrichtung mit Bezugszeichen 305a bezeichnet, die der Fluidleitung 322 zugeordnete Luftmassenmessvorrichtung mit Bezugszeichen 305b und die der Fluidleitung 321 zugeordnete Luftmassenmessvorrichtung mit Bezugszeichen 305d. Alternativ oder zusätzlich kann auch im Ventil 120 selbst der Messdraht einer Luftmassenmessvorrichtung angeordnet sein. In Fig. 8 ist diese Luftmassenmessvorrichtung symbolisch mit dem Bezugszeichen 305c bezeichnet. Bei einem Sitzkomfortsystem 2 mit mehreren Luftkissen 330 können die mehreren Luftkissen 330 mit einem oder mehreren Ventilen 120 verbunden sein. Insbesondere sind mehrere Luftkissen 330 mit einer Ventilanordnung 200 verbunden, wobei eine Ventilanordnung 200, beispielsweise eine Ventilanordnung nach Fig. 7, im Wesentlichen das in Fig. 8 dargestellte Ventil 120 ersetzen würde, und statt dem einen gezeigten Luftkissen 330 mehrere Luftkissen 330 parallel mit der Ventilanordnung 200 verbunden wären. Die Luftmassenmessvorrichtungen, insbesondere die Messdrähte der Luftmassenmessvorrichtungen, können dann entsprechend in der Ventilanordnung 200 und/oder den jeweiligen Luftkanälen und/oder Strömungsbereichen und/oder Fluidleitungen angeordnet sein.

### Bezugszeichenliste

1 Schaltungsanordnung
2 Sitzkomfortsystem
4 ASIC
5 Messvorrichtung
6 Treibereinheit
8 Auswerteeinheit
12 Multiplexer
13 Stromquelle
14 Signalverstärker
16 Filter
20, 20-1 bis 20-N SMA-Treiber
21-1 bis 21-N Vorwiderstand
23-1 bis 23-M Vorwiderstand
30 Steuereinheit
31 Eingang
36 Speicher
38 Feedback-Vorrichtung
60 Pulsweitenmodulationsvorrichtung
70 Temperatursensor
71 Spannungssensor
100, 100-1 bis 100-N SMA-Element, beispielsweise SMA-Draht
101 Crimp
102 Ventilgehäuse
103 Aktuator
104 Stellelement
104a Durchloch
105 erste Öffnung
106 zweite Öffnung
107 Endschalter
108 Dichtelement
109 Ventilraum
110 Leiterplatte
111 Grundplatte
120, 120a, 120b Ventil
140, 140-1 bis 140-M Draht
141 bis 146 Draht
200 Ventilanordnung
208 Zwischengehäuse
230a bis 230d Verbraucheranschluss
270 Druckanschluss
272 Rückschlagventil
274 zweiter Strömungsbereich
276 Luftkanal
278a bis 278d Luftkanal
282 erster Strömungsbereich
300 Pumpe
305-1 bis 205-N, 305-X Luftmassenmessvorrichtung
305a bis 305d Luftmassenmessvorrichtung
310 erste Ventilöffnung
311 zweite Ventilöffnung
312 dritte Ventilöffnung
320 Fluidleitung
321 Fluidleitung
322 Fluidleitung
330 Luftkissen
340 Atmosphärenöffnung
U Spannungsquelle

## Patentansprüche

1. Sitzkomfortsystem (2) umfassend
ein oder mehrere Luftkissen (330), wobei der Füllstand jedes Luftkissens (330) über zumindest ein Ventil (120) steuerbar ist, und
ein oder mehrere Schaltungsanordnungen (1) zur Ansteuerung des oder der Ventile (120),
wobei das mindestens eine Ventil zumindest einen Aktuator (103) mit zumindest einem Stellelement (104) umfasst,
wobei das Stellelement (104) zumindest zwischen einer ersten Stellung und einer zweiten Stellung verstellbar ist,
wobei die Schaltungsanordnung (1) zumindest eine Treibereinheit (6) zum Betätigen des Aktuators (103) und eine Steuereinheit (30) zum Steuern der Treibereinheit (6) aufweist,
**dadurch gekennzeichnet, dass**
die Schaltungsanordnung (1) zumindest eine Luftmassenmessvorrichtung (305-1 bis 305-N, 305-X, 305a bis 305d) zur Messung einer das Ventil durchströmenden Luftmasse umfasst,
wobei die Steuereinheit (30) dazu geeignet ist, ein Ausgangssignal der Luftmassenmessvorrichtung (305-1 bis 305-N, 305-X, 305a bis 305d) zu verarbeiten.

2. Sitzkomfortsystem (2) nach Anspruch 1, wobei die Luftmassenmessvorrichtung (305-1 bis 305-N, 305-X, 305a bis 305d) zumindest einen Messdraht (100, 100-1 bis 100-N, 140, 140-1 bis 140-M) und eine Messvorrichtung (5) umfasst, wobei die Messvorrichtung (5)
a. eine Widerstandsmessvorrichtung zur Messung des Widerstands des Messdrahtes und/oder
b. einen Temperatursensor und/oder eine Temperaturmessvorrichtung zur Messung einer Temperatur des Messdrahtes und/oder der Umgebungstemperatur des Messdrahtes und/oder
c. eine Strommessvorrichtung zur Messung eines Heizstroms des Messdrahtes und/oder
d. eine Leistungsmessvorrichtung zur Messung einer Eingangsleistung des Messdrahtes umfasst.

3. Sitzkomfortsystem (2) nach Anspruch 2, wobei die Luftmassenmessvorrichtung (305-1 bis 305-N, 305-X, 305a bis 305d) und/oder die Steuereinheit (30) dazu eingerichtet sind, die Luftmasse aus
a. der Temperatur des Messdrahtes und dem Heizstrom und/oder
b. der Temperatur des Messdrahtes und der Eingangsleistung und/oder
c. dem Widerstand des Messdrahtes und dem Heizstrom und/oder
d. dem Widerstand des Messdrahtes und der Eingangsleistung zu ermitteln.

4. Sitzkomfortsystem (2) nach einem der vorhergehenden Ansprüche, wobei der Aktuator (103) ein Shape Memory Alloy-Element (SMA-Element) (100, 100-1 bis 100-N) umfasst.

5. Sitzkomfortsystem (2) nach Anspruch 4,
wobei das SMA-Element (100, 100-1 bis 100-N) ein SMA-Draht ist,
wobei die Luftmassenmessvorrichtung (305-1 bis 305-N, 305-X, 305a bis 305d) zumindest einen Messdraht (100, 100-1 bis 100-N) umfasst,
wobei der SMA-Draht der Messdraht oder einer der Messdrähte der Luftmassenmessvorrichtung ist.

6. Sitzkomfortsystem (2) nach Anspruch 5,
wobei die Luftmassenmessvorrichtung (305-1 bis 305-N, 305-X, 305a bis 305d) eine Messvorrichtung (5) umfasst,
wobei die Schaltungsanordnung (1) dazu ausgelegt ist, dass die Messvorrichtung (5) und die Treibereinheit (6) alternierend mit dem SMA-Element (100, 100-1 bis 100-N) wirkverbunden sind.

7. Sitzkomfortsystem (2) nach einem der Ansprüche 4 bis 6,
wobei die Luftmassenmessvorrichtung (305-1 bis 305-N, 305-X, 305a bis 305d) zumindest einen Messdraht (140, 140-1 bis 140-M) umfasst,
wobei zusätzlich zum SMA-Element zumindest ein separater Draht (140, 140-1 bis 140-M) vorgesehen ist,
wobei der separate Draht der Messdraht oder einer der Messdrähte der Luftmassenmessvorrichtung ist.

8. Sitzkomfortsystem (2) nach einem der vorhergehenden Ansprüche, wobei die Schaltungsanordnung (1) einen ASIC (4) umfasst, welcher eine oder mehrere der folgenden Komponenten aufweist: die Treibereinheit (6), eine Messvorrichtung (5) der Luftmassenmessvorrichtung (305-1 bis 305-N, 305-X, 305a bis 305d) und die Steuereinheit (30).

9. Sitzkomfortsystem (2) nach einem der vorhergehenden Ansprüche, wobei das oder die Ventile (120) ein Ventilgehäuse (102) aufweisen, wobei das Ventilgehäuse (102) zumindest eine erste Öffnung (105) und eine zweite Öffnung (106) aufweist,
wobei das Ventilgehäuse (102) einen Ventilraum (109) umschließt, und wobei das Stellelement (104) zum Öffnen oder Schließen des Ventils (120) angeordnet ist.

10. Sitzkomfortsystem (2) nach Anspruch 9, wobei ein Messdraht (100, 100-1 bis 100-N, 140, 140-1 bis 140-M) der Luftmassenmessvorrichtung (305-1 bis 305-N, 305-X, 305a bis 305d) der Schaltungsanordnung (1) im Ventilraum (109) des Ventils (120) angeordnet ist.

11. Sitzkomfortsystem (2) nach Anspruch 9 oder 10, wobei mehrere der Ventile (120) vorgesehen sind, die eine Ventilanordnung bilden, wobei die Schaltungsanordnung (1) zur Steuerung jedes Ventils (120) in eine gemeinsame Schaltungsanordnung integriert ist.

## Claims

1. Seat comfort system (2) comprising
one or more air cushions (330), the filling level of each air cushion (330) being controllable via at least one valve (120), and
one or more circuit arrangements (1) for controlling the valve or valves (120), wherein the at least one valve comprises at least one actuator (103) with at least one actuating element (104),
wherein the actuating element (104) is adjustable at least between a first position and a second position,
wherein the circuit arrangement (1) has at least one driver unit (6) for actuating the actuator (103) and a control unit (30) for controlling the driver unit (6),
**characterised in that**
the circuit arrangement (1) comprises at least one air mass measuring device (305-1 to 305-N, 305-X, 305a to 305d) for measuring an air mass flowing through the valve,
wherein the control unit (30) is suitable for processing an output signal of the air mass measuring device (305-1 to 305-N, 305-X, 305a to 305d).

2. The seat comfort system (2) according to claim 1, wherein the air mass measuring device (305-1 to 305-N, 305-X, 305a to 305d) comprises at least one measuring wire (100, 100-1 to 100-N, 140, 140-1 to 140-M) and a measuring device (5), wherein the measuring device (5) comprises
a. a resistance measuring device for measuring the resistance of the measuring wire and/or
b. a temperature sensor and/or a temperature measuring device for measuring a temperature of the measuring wire and/or the ambient temperature of the measuring wire and/or
c. a current measuring device for measuring a heating current of the measuring wire and/or
d. comprises a power measuring device for measuring an input power of the measuring wire.

3. Seat comfort system (2) according to claim 2, wherein the air mass measuring device (305-1 to 305-N, 305-X, 305a to 305d) and/or the control unit (30) are set up to measure the air mass from
a. the temperature of the measuring wire and the heating current and/or
b. the temperature of the measuring wire and the input power and/or
c. the resistance of the measuring wire and the heating current and/or
d. the resistance of the measuring wire and the input power can be determined.

4. A seat comfort system (2) according to any one of the preceding claims,
wherein the actuator (103) comprises a Shape Memory Alloy element (SMA element) (100, 100-1 to 100-N).

5. Seat comfort system (2) according to claim 4,
wherein the SMA element (100, 100-1 to 100-N) is an SMA wire,
wherein the air mass measuring device (305-1 to 305-N, 305-X, 305a to 305d) comprises at least one measuring wire (100, 100-1 to 100-N),
wherein the SMA wire is the measuring wire or one of the measuring wires of the air mass measuring device.

6. Seat comfort system (2) according to claim 5,
wherein the air mass measuring device (305-1 to 305-N, 305-X, 305a to 305d) comprises a measuring device (5),
wherein the circuit arrangement (1) is designed so that the measuring device (5) and the driver unit (6) are operatively connected alternately to the SMA element (100, 100-1 to 100-N).

7. Seat comfort system (2) according to one of claims 4 to 6,
wherein the air mass measuring device (305-1 to 305-N, 305-X, 305a to 305d) comprises at least one measuring wire (140, 140-1 to 140-M),
wherein at least one separate wire (140, 140-1 to 140-M) is provided in addition to the SMA element,
wherein the separate wire is the measuring wire or one of the measuring wires of the air mass measuring device.

8. Seat comfort system (2) according to one of the preceding claims, wherein the circuit arrangement (1) comprises an ASIC (4) which has one or more of the following components: the driver unit (6), a measuring device (5) of the air mass measuring device (305-1 to 305-N, 305-X, 305a to 305d) and the control unit (30).

9. Seat comfort system (2) according to any one of the preceding claims, wherein the valve or valves (120) comprise a valve housing (102), wherein the valve housing (102) comprises at least a first opening (105) and a second opening (106),
wherein the valve housing (102) encloses a valve chamber (109), and wherein the actuating element (104) is arranged to open or close the valve (120).

10. Seat comfort system (2) according to claim 9, wherein a measuring wire (100, 100-1 to 100-N, 140, 140-1 to 140-M) of the air mass measuring device (305-1 to 305-N, 305-X, 305a to 305d) of the circuit arrangement (1) is arranged in the valve chamber (109) of the valve (120).

11. Seat comfort system (2) according to claim 9 or 10, wherein several of the valves (120) are provided, which form a valve arrangement, wherein the circuit arrangement (1) for controlling each valve (120) is integrated into a common circuit arrangement.

## Revendications

1. Système de confort de siège (2) comprenant
un ou plusieurs coussins d'air (330), le niveau de remplissage de chaque coussin d'air (330) pouvant être commandé par au moins une soupape (120), et un ou plusieurs dispositifs de circuit (1) pour la commande de la ou des soupapes (120),
l'au moins une soupape comprenant au moins un actionneur (103) avec au moins un élément de réglage (104),
l'élément de réglage (104) étant réglable au moins entre une première position et une deuxième position,
le circuit (1) présentant au moins une unité d'entraînement (6) pour actionner l'actionneur (103) et une unité de commande (30) pour commander l'unité d'entraînement (6),
**caractérisé en ce que**
le circuit (1) comprend au moins un dispositif de mesure de la masse d'air (305-1 à 305-N, 305-X, 305a à 305d) pour mesurer une masse d'air traversant la soupape,
dans lequel l'unité de commande (30) est adaptée pour traiter un signal de sortie du dispositif de mesure de la masse d'air (305-1 à 305-N, 305-X, 305a à 305d).

2. Système de confort de siège (2) selon la revendication 1, dans lequel le dispositif de mesure de la masse d'air (305-1 à 305-N, 305-X, 305a à 305d) comprend au moins un fil de mesure (100, 100-1 à 100-N, 140, 140-1 à 140-M) et un dispositif de mesure (5), dans lequel le dispositif de mesure (5)
a. un dispositif de mesure de la résistance pour mesurer la résistance du fil de mesure et/ou
b. un capteur de température et/ou un dispositif de mesure de la température pour mesurer une température du fil de mesure et/ou la température ambiante du fil de mesure et/ou
c. un dispositif de mesure du courant pour mesurer un courant de chauffage du fil de mesure et/ou
d. comprend un dispositif de mesure de puissance pour mesurer une puissance d'entrée du fil de mesure.

3. Système de confort de siège (2) selon la revendication 2, dans lequel le dispositif de mesure de la masse d'air (305-1 à 305-N, 305-X, 305a à 305d) et/ou l'unité de commande (30) sont conçus pour déterminer la masse d'air à partir de
a. la température du fil de mesure et le courant de chauffage et/ou
b. de la température du fil de mesure et de la puissance d'entrée et/ou
c. la résistance du fil de mesure et le courant de chauffage et/ou
d. la résistance du fil de mesure et la puissance d'entrée à déterminer.

4. Système de confort de siège (2) selon l'une quelconque des revendications précédentes, dans lequel l'actionneur (103) comprend un élément en alliage à mémoire de forme (SMA) (100, 100-1 à 100-N).

5. Système de confort de siège (2) selon la revendication 4,
dans lequel l'élément SMA (100, 100-1 à 100-N) est un fil SMA,
le dispositif de mesure de la masse d'air (305-1 à 305-N, 305-X, 305a à 305d) comprenant au moins un fil de mesure (100, 100-1 à 100-N),
le fil SMA étant le fil de mesure ou l'un des fils de mesure du dispositif de mesure de la masse d'air.

6. Système de confort de siège (2) selon la revendication 5,
dans lequel le dispositif de mesure de la masse d'air (305-1 à 305-N, 305-X, 305a à 305d) comprend un dispositif de mesure (5),
l'agencement de circuit (1) étant conçu pour que le dispositif de mesure (5) et l'unité d'entraînement (6) soient reliés fonctionnellement en alternance à l'élément SMA (100, 100-1 à 100-N).

7. Système de confort de siège (2) selon l'une des revendications 4 à 6,
dans lequel le dispositif de mesure de la masse d'air (305-1 à 305-N, 305-X, 305a à 305d) comprend au moins un fil de mesure (140, 140-1 à 140-M), au moins un fil séparé (140, 140-1 à 140-M) étant prévu en plus de l'élément SMA,
le fil séparé étant le fil de mesure ou l'un des fils de mesure du dispositif de mesure de la masse d'air.

8. Système de confort de siège (2) selon l'une des revendications précédentes, dans lequel l'agencement de circuit (1) comprend un ASIC (4) qui présente un ou plusieurs des composants suivants : l'unité de commande (6), un dispositif de mesure (5) du dispositif de mesure de la masse d'air (305-1 à 305-N, 305-X, 305a à 305d) et l'unité de commande (30).

9. Système de confort de siège (2) selon l'une quelconque des revendications précédentes, dans lequel la ou les valves (120) comprennent un corps de valve (102), le corps de valve (102) comprenant au moins une première ouverture (105) et une deuxième ouverture (106),
le boîtier de soupape (102) entourant une chambre de soupape (109), et l'élément de réglage (104) étant disposé pour ouvrir ou fermer la soupape (120).

10. Système de confort de siège (2) selon la revendication 9, dans lequel un fil de mesure (100, 100-1 à 100-N, 140, 140-1 à 140-M) du dispositif de mesure de la masse d'air (305-1 à 305-N, 305-X, 305a à 305d) du circuit (1) est disposé dans la chambre de soupape (109) de la soupape (120).

11. Système de confort de siège (2) selon la revendication 9 ou 10, dans lequel plusieurs des soupapes (120) sont prévues, qui forment un agencement de soupapes, l'agencement de circuit (1) pour la commande de chaque soupape (120) étant intégré dans un agencement de circuit commun.
